# EUROPEAN PATENT APPLICATION

(11) **EP 4 459 273 A1**
(43) Date of publication of application: **06.11.2024**
(21) Application number: 23171367.8
(22) Date of filing: 03.05.2023
(51) Int. Cl.: G01N 27/327, C12Q 1/26

(54) **A METHOD FOR OBTAINING AN ENZYME ELECTRODE WITH A MEMBRANE FOR DETECTION AND QUANTIFICATION OF L-AMINO ACIDS AND ENZYME ELECTRODE WITH A MEMBRANE FOR DETECTION AND QUANTIFICATION OF L-AMINO ACIDS**

(71) Applicant: Vilnius University, 01513 Vilnius (LT)
(72) Inventor: Miskinis, Justas, Vilnius (LT); Ramonas, Eimantas, Vilnius (LT); Stakelyte, Deimante, Vilnius (LT); Gureviciene, Vidute, Vilnius (LT); Razumiene, Julija, Vilnius (LT); Dagys, Marius, Vilnius (LT); Ratautas, Dalius, Vilnius (LT)
(74) Representative: Pakeniene, Ausra

(57) **Abstract**

A method for preparation of an enzyme electrode comprising a membrane where the membrane is directly synthesized on the electrode surface suitable for conducting oxidation of L-amino acids and resulting in detection and quantification of L-amino acids from complex biological samples such as human serum and blood. The method for the membrane synthesis directly on the electrode surface comprises preparing electrode surface comprising gold nanoparticles or similar nanostructures ensuring high electrode cleanness for creating high surface area for optimal reaction conditions with a membrane composed of sulfonated tetrafluoroethylene based fluoropolymer-copolymer protective membrane for removal of interfering substances and synthesizing enzymatic membrane containing L-amino acid oxidase, bovine serum albumin and glutaraldehyde as crosslinker directly on the surface of the electrode.

## Description

### Technical Field

Invention is related to enzyme electrodes and in particular enzyme electrodes with membranes for the detection and quantification of L-amino acids.

### Background Art

Detection and measurement of L-amino acids is important for clinical and healthcare purposes in general. L-amino acids can be detected in complex samples such as human serum and blood. In clinical diagnostics L-amino acids are important and are related with many various conditions such as inherited metabolic disorders, e.g. maple syrup urine disease [1], dyslipidemia [2][3], type 2 diabetes [4], muscle condition, cancer [6], cardiovascular disease [7], septic shock [8] and various mental disorders [9]. To add more, recently the importance of monitoring and measurements of L-amino acids have risen for patients undergoing renal replacement therapy in the intensive care units, since it was reported that those patients can lose as much as 5-15 g of L-amino acids per day. A significant variety of clinical conditions related to L-amino acids indicate, that measurement of L-amino acids is needed. However, the detection of L-amino acids is difficult and, typically, is not a routine procedure.

Currently L-amino acids are being measured using various methods such as high-performance liquid chromatography or amino acid analyzers. Electrochemical methods could be an attractive, cheaper, and faster alternative method to measure L-amino acids in biological samples. For example, biosensors were successfully designed and applied for measurements in various clinically relevant analytes such as glucose, formaldehyde, glycerol, lactate and others. The number of biosensors and enzyme membrane electrodes suitable to measure is limited.

European patent No.: EP2163888B1 discloses a design of an enzyme electrode and enzyme sensor where enzyme was immobilized in mesoporous silica material provided on the electrode. The key drawback of the invention is the requirement to use an electron carrier to facilitate transfer of an electron between the enzyme and the electrode.

US patent No.: US7455874B2 discloses a method for fabrication of biosensors comprising enzymes involving surfaces with specific roughness with additional semipermeable membrane. The key drawback of the invention is the requirement to use complex curing procedures to form substrate with suitable surface roughness.

US patent No.: US9441259B2 discloses a method for development of enzyme compositions on an electrode using various crosslinkers activated by UV. The key drawback of the invention is the requirement to use complex curing procedures to form substrate with suitable surface roughness.

US patent No.: US11134874B2 discloses a working electrode with a carbon-enzyme layer. Sensory layer was obtained by mixing an aqueous polyurethane emulsion with acrylic polyol emulsion followed by the addition of enzyme and carbon-based material. The sensor electrode was used for continuous biological monitoring. The key drawback of the invention is the requirement to make a complex carbon-enzyme layer involving several organic compounds to form the emulsion.

US patent No.:US9388503B2 discloses an electrochemical sensor system, membrane and method for detection of glucose and lactate. The electrode was designed using glucose and/or lactate oxidase(s) immobilized on Pt electrode covered with protective membrane. The key drawback of the invention is the requirement to conduct electrochemical polymerization.

None of the prior art discloses determination and/or detection of amino acids.

It was demonstrated that enzyme electrode can be constructed by placing a prepared enzyme polymer on a platinum disk surface and covering using nylon cloth [10].

A biosensor for the measurement of total loss of L-amino acids was also reported based on platinum-gold nanoparticle electrode covered with enzymatic membrane [11].

The present invention is dedicated to overcoming the above shortcomings and for producing further advantages over prior art.

### Brief description of the invention

The invention discloses a method for preparation of an enzyme electrode comprising a membrane where the membrane is directly synthesized on the electrode surface suitable for conducting oxidation of L-amino acids and resulting in detection and quantification of L-amino acids from complex biological samples such as human serum and blood.

According to one aspect of the invention method for the membrane synthesis directly on the electrode surface comprises the following steps: (i) preparing electrode surface comprising gold nanoparticles or similar nanostructures ensuring high electrode cleanness, i.e. free of molecules which could result in electrochemical interference, for (ii) creating high surface area for optimal, i.e. free of electrochemical interference, reaction conditions with a membrane composed of sulfonated tetrafluoroethylene based fluoropolymer-copolymer, for example *Nafion* or similar in principle suitable to impede transfer of anions, protective membrane for removal of interfering substances and (iii) synthesizing enzymatic membrane containing L-amino acid oxidase, bovine serum albumin and glutaraldehyde as crosslinker directly on the surface of the electrode.

According to another aspect of the invention an enzyme electrode with a membrane suitable for detection and quantification of L-amino acids comprising enzymatic membrane with crosslinked L-amino acid oxidase is disclosed. The electrode surface comprises gold nanoparticles or similar nanostructures with a *Nafion-based* or similar in function protective membrane for removal of interfering substances forms a reactive surface which has a high surface area and can oxidize hydrogen peroxide. As the result, hydrogen peroxide can be oxidized and electrons are transferred to the current-potential measuring device allowing to relate the device output signal with the initial concentration of L-amino acids. *Nation-*based or similar protective membrane is required to remove the interference which could cause elevated or decreased signal. The protective membrane allows diffusion of hydrogen peroxide while impeding the movement of high molecular mass particles and negatively charged ions. Afterwards, the synthesized enzymatic membrane containing L-amino acid oxidase, bovine serum albumin and crosslinker glutaraldehyde is required to conduct a biochemical reaction for a specific oxidation of L-amino acids and production of reaction intermediate hydrogen peroxide.

### Brief description of the drawings

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:

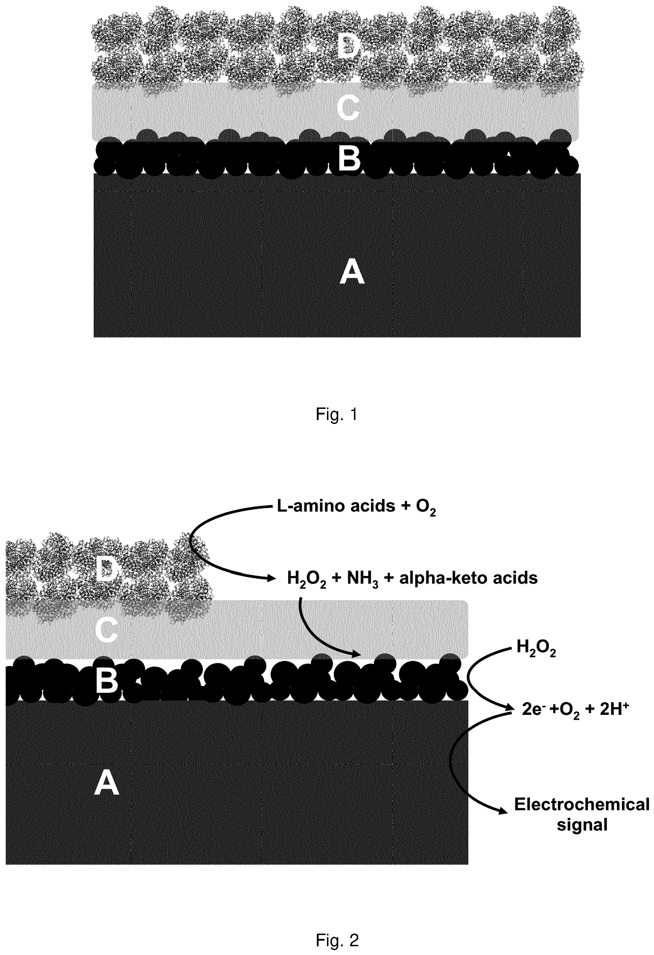

Fig. 1 shows a scheme of the enzyme electrode according to the invention, where: A - base of the electrode, made from gold, platinum, carbon or other material with similar chemical and physical properties; B - nanoparticle layer, made of gold, platinum, silver, carbon or other nanoparticles with similar chemical and physical properties; C - protective membrane composed of sulfonated tetrafluoroethylene based fluoropolymer-copolymer, for example *Nafion* or similar in principle suitable to impede the transfer of anions, for removal of interfering substances; D - enzyme layer comprising L-amino acid oxidase, bovine serum albumin and crosslinking chemical glutaraldehyde.
Fig. 2 shows a scheme of operation principle of the electrode with the enzyme membrane according to the invention, where: D - enzyme layer, comprising L-amino acid oxidase, bovine serum albumin and crosslinking chemical glutaraldehyde, catalyze the oxidation of L-amino acids with the molecular oxygen (O₂) to form hydrogen peroxide (H₂O₂), ammonia (NH₃) and alpha-keto acids; C - protective membrane, as described above, for removing the interference by allowing the transport of H₂O₂ towards the reactive surface while significantly impeding the transfer of negatively charged ions; B - nanoparticle, such as gold, platinum, silver, carbon or similar nanoparticle layer, as describe above, forming a reactive surface catalyzing H₂O₂ oxidation forming O₂, protons and electrons. Received formed electrons, i.e. electric current, are directly proportional to the activity of L-amino acid oxidase and in turn to the concentration of L-amino acids in solution; A - base of the electrode, made from gold, platinum, carbon or similar material, as described above.
Fig. 3 shows electrode calibration using L-leucine. The black curve represents data fitting using Michaelis-Menten model.
Fig. 4 shows calibration of the electrode using analytical L-amino acid standard. The black curve represents data fitting using Michaelis-Menten model.
Fig. 5 shows calibration of the electrode using L-amino acid standard, containing 15 L-amino acids as described in Table 1. The black curve represents the data fitting using Michaelis-Menten model.
Fig. 6 shows calibration of the electrode using analytical L-amino acid standard and coulometric measurement method. The black line represents the data fitting using linear model.

### Detailed description of the Invention

The invention is a preparation method of an enzyme electrode comprising enzymatic membrane with crosslinked L-amino acid oxidase comprising the following steps: (i) preparing a catalytically reactive surface of the electrode, the catalytically reactive surface comprising gold nanoparticles, or other nanostructures having similar chemical and physical properties, ensuring high electrode cleanness, i.e. free of molecules which could result in electrochemical interference, for (ii) creating high surface area for optimal, i.e. free of electrochemical interference, reaction conditions with a membrane composed of sulfonated tetrafluoroethylene based fluoropolymer-copolymer, for example *Nafion* or similar in principle suitable to impede the transfer of anions, protective membrane for removal of interfering substances and (iii) synthesizing enzymatic membrane containing L-amino acid oxidase, bovine serum albumin and glutaraldehyde as crosslinker directly on the surface of the electrode.

The catalytically reactive surface of the electrode comprises gold nanoparticles or other nanostructures having similar chemical and physical properties for creating high reactive surface for optimal, i.e. free of electrochemical interference, reaction conditions. The electrode made from highly conductive material, selected from metal, such as gold, platinum, palladium, silver, nickel, antimony, titanium, ruthenium, iridium, rhodium, steel, copper or other metal having similar chemical or physical properties, or carbon, such as carbon particles, graphite, graphene, carbon nanotubes, fullerene-based glassy carbon or other carbon isoform having similar chemical of physical properties. The electrode surface is thoroughly cleaned prior the formation of the catalytically reactive surface to ensure the removed of redox-active contaminants from the electrochemical surface. Cleaning is performed by polishing the electrode using ultra-fine emery paper following polishing with polishing mixture containing microparticles or nanoparticles, for example aluminum oxide, diamond or similar. Afterwards the surface is thoroughly cleaned by placing the electrode into an ultrasound bath and applying ultrasound treatment to remove any left contaminants.

In a preferred embodiment, the catalytically reactive surface is formed by adding a layer capable of increasing the surface area and to catalyze the oxidation of hydrogen peroxide to oxygen in an electrochemical potential range from -0.4 V to 0.6 V vs. SHE (standard hydrogen electrode). This catalytically reactive surface layer is made using chemically reactive nanoparticles or microparticles of 2-2000 nm range in diameter made from metallic nanoparticles selected from gold, platinum, palladium, silver, nickel, antimony, titanium, ruthenium, iridium, rhodium, steel, copper or other material having similar chemical and physical properties; carbon material selected from carbon particles, graphite, graphene, carbon nanotubes, fullerene-based particles or other form of carbon having similar chemical and physical properties; or composite material selected from organic polymers, carbon-metallic particles, bimetallic particles or other form of carbon having similar chemical and physical properties.

In the preferred embodiment, a solution of 2.0-10.0 µL volume containing gold nanoparticles, having diameter of 17 nm and molar concentration of 0.27 µmol/L is drop-casted on of the electrode surface and allowed to dry. Dried gold nanoparticles form the reactive surface which is capable to chemically oxidize hydrogen peroxide at high rate. Afterwards, the surface is covered using a *Nafion-based* or similar, in principle suitable to impede the transfer of anions, membrane for removal of interfering substances. The protective membrane is formed by directly placing solution or gel on the reactive surface and allowing it to dry. As an example, the protective membrane is formed by placing 2.0 µL of *Nation* solution, concentration 0.05% V/V, directly of the reactive surface. The protective membrane can also be selected from, cellulose-, polydimethylsiloxane-, poly(vinyl chloride)-, polyethylene-, Teflon-, PTFE- or other material having similar chemical and physical properties. The solution is allowed to dry and fully cover the reactive surface. Afterwards, an enzymatic membrane is formed on the *Nafion-based* or similar material based membrane. The enzymatic membrane is formed using solutions containing L-amino acid oxidase, bovine serum albumin or other protein having similar chemical or physical properties and glutaraldehyde or other compound having similar chemical or physical properties selected from carbodiimides (EDC and/or DCC), N-hydroxysuccinimide esters (e.g., NHS), imidoesters, maleimides, haloacetyls, pyridyl disulfides, hydrazides. Concentration of the solutions of L-amino acid oxidase, bovine serum albumin and glutaraldehyde are 50 mg/mL, 40 mg/mL and 1.0%, respectively. The enzymatic membrane in the preferred embodiment is prepared by mixing of 14.0 µL L-amino acid oxidase solution with 6.0 µL of bovine serum albumin solution in a test-tube taking care that bubbles do not form. Afterwards, the received solution, around 20 µL is placed on the *Nafion-based* membrane covered reactive surface, equally distributed and 4.0 µL of glutaraldehyde solution is placed on top. Final steps involve drying the electrode to form a solid membrane of the reactive surface. In the preferred embodiment the electrode is dried for 15 min. after the addition of 4.0 µL of glutaraldehyde solution, at 0°C-50°C temperature. Afterwards the electrode is transferred and dried for 24h at high humidity environment, for example 70-100%, such as enclosed water bath, at room temperature, for example 18°C-30°C.

Examples of testing and use of electrodes obtained by the method according to the invention:

### Example 1: Quantification of L-Leucine concentration in buffer solution

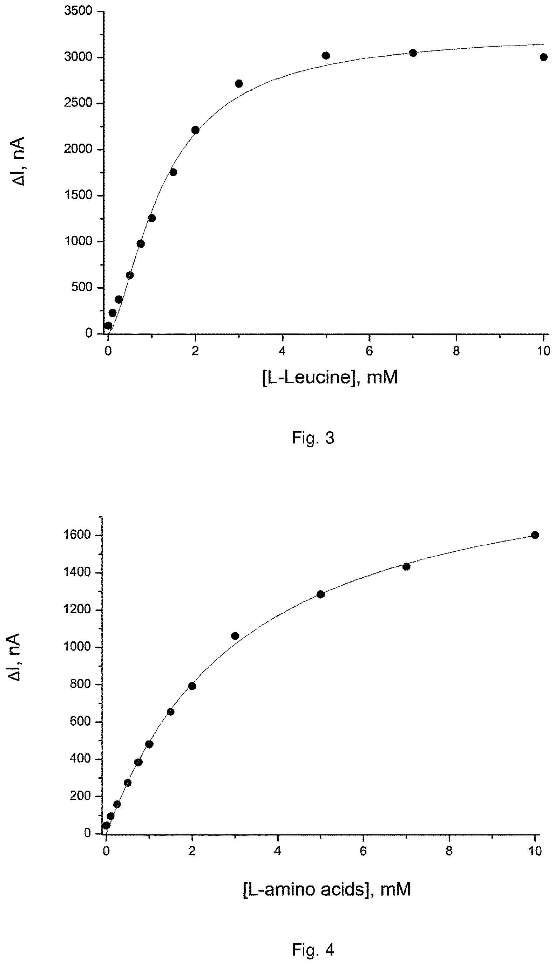

An electrode prepared according to method of the invention was tested to measure L-leucine concentration in a working buffer solution (WBS), containing 50 mM K₂HPO₄ and 100 mM NaCl, and the pH was adjusted to 7.2 using HCl. Electrode potential was set to 400 mV vs. Ag/AgCI reference electrode. Measurements were performed by adding a certain concentration of L-leucine and measuring the electrochemical response (Figure 3).

### Example 2: Detection of total L-amino acids in buffer solution

An electrode obtained by the method according to the invention was tested to measure total L-amino acid concentration. The electrode was calibrated using an analytical L-amino acid standard solution (purchased from Sigma-Aldrich, SKU AAS18-10ML), which contains 17 common L-amino acids, each with a concentration of 2.5 mM (except L-Cystine at 1.25 mM) (Figure 4). The electrode demonstrated a current increase depending on the concentration of analytical L-amino acid standard solution.

### Example 3: Detection of total amino acids using modified L-amino acid standard

To evaluate sensor comprising an electrode obtained by the method according to the invention, Ag/AgCI (3M KCI) reference electrode, platinum counter electrode and standard electrochemical workstation suitable to conduct amperometric analysis performance in measuring total amino acids in bodily fluids, L-amino acid standard, containing 15 L-amino acids was prepared (Table 1).

**Table 1. The L-amino acid concentration in the calibration solution**

| **L-amino acid** | **[L-amino acid], mM** |
|---|---|
| L-Alanine | 17.12 |
| L-Glutamine | 15.20 |
| L-Valine | 9.28 |
| L-Glycine | 9.06 |
| L-Glutamic acid | 8.90 |
| L-Threonine | 6.52 |
| L-Lysine | 6.21 |
| L-Leucine | 5.59 |
| L-Isoleucine | 4.69 |
| L-Arginine | 4.27 |
| L-Phenylalanine | 3.21 |
| L-Tyrosine | 3.14 |
| L-Histidine | 2.60 |
| L-Methionine | 2.19 |
| L-Tryptophane | 2.03 |

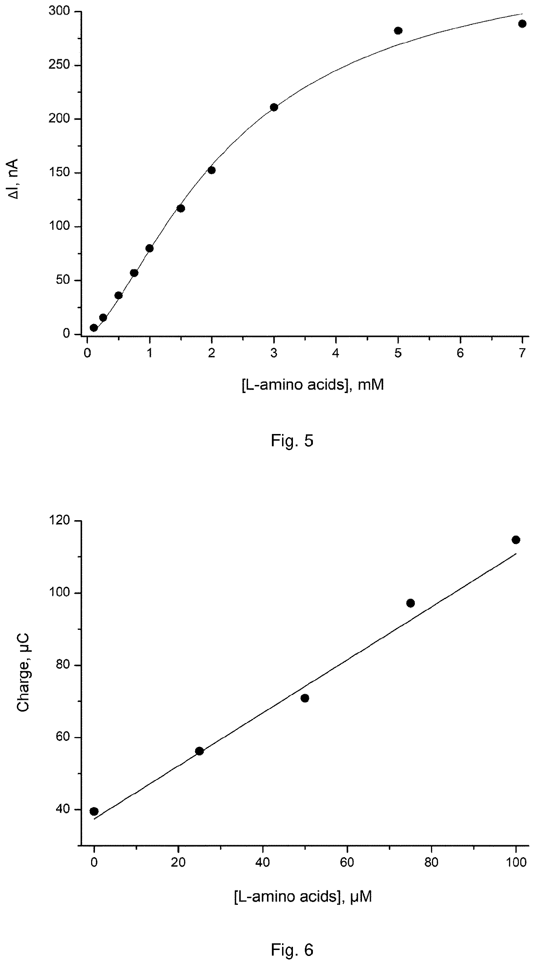

The sensor comprising the electrode according to the invention was calibrated using an L-amino acid standard (Figure 5), containing 15 L-amino acids with concentrations proportional to those, that are usually found in healthy human individuals' blood (Krebs, 1950). The concentration of total L-amino acids in this calibrating solution was 100 mM.

### Example 4: Quantification of relative loss of L-amino acids in human blood

The sensor as defined in Example 3 was tested to measure total L-amino acid concentration loss in human blood during hemodialysis. Before the measurements, the electrode was calibrated using an analytical L-amino acid standard and the obtained calibration curve was used to calculate L-amino acid concentration in blood samples.

Eighteen blood samples were taken from nine patients, undergoing renal replacement therapy. The first samples (A samples) were taken from the blood before entering the hemodialysis apparatus, while the second blood samples (B samples) were taken from the blood leaving the apparatus (Table 2). 50 µL of raw serum was added to a 1000 µL electrochemical cell containing a buffer solution to take measurements.

**Table 2. Measurement of total L-amino acid concentration in human blood serum**

| **Patient code** | **[total L-amino acids], mM** |
|---|---|
| P31_A1 | 2.84 |
| P31_V1 | 1.74 |
| P31_A2 | 3.78 |
| P31_V2 | 2.27 |
| P31_A3 | 2.18 |
| P31_V3 | 1.77 |
| P18_A1 | 5.19 |
| P18_V1 | 3.51 |
| P25_A1 | 6.28 |
| P25_V1 | 3.81 |
| P27_A3 | 5.99 |
| P27_V3 | 2.72 |
| P23_A3 | 2.99 |
| P23_V3 | 2.09 |
| P21_A1 | 3.19 |
| P21_V1 | 2.97 |
| P22_A4 | 4.95 |
| P22_V4 | 2.26 |

As the results of the measurements show, the sensor of the Example 3 is capable to measure the L-amino acid concentration difference between samples, taken before and after hemodialysis apparatus, thus can be applied to evaluate the rate of amino acid concentration decrease in patients, undergoing renal replacement therapy.

### Example 5: Quantification of total L-amino acids in human blood using the coulometric method

To increase sensitivity and achieve a lower detection limit, coulometric (capacitance) measurements were conducted by using multistep amperometry. The measurement algorithm consisted of potential-current steps. At first, the electrode prepared according to method according to the invention was polarized at 400 mV without the analyte (L-amino acids) allowing the electrode surface to fully discharge. Subsequently, the electrode was disconnected from the circuit and thus left polarized at a residual potential of about the same 400 mV value after the addition of the analytes for 100 s. Once the analytes were added, the discharged electrode was oxidized and accumulated the electric charge on the surface. Subsequently, the electrode was connected to a circuit, and the potential-current step was conducted once again to polarize the electrode to 400 mV. The current flowing to the electrode was recorded for 2000 ms and was used to calculate the total electrode charge.

The electrode was calibrated using the analytical L-amino acid standard (Figure 6).

For clarity and sufficiency of disclosure the term *Nafion* is herewith specified as:
a brand name for a sulfonated tetrafluoroethylene based fluoropolymer-copolymer. *Nafion's* unique ionic properties are a result of incorporating perfluorovinyl ether groups terminated with sulfonate groups onto a tetrafluoroethylene (PTFE) backbone. Ion conductivity of *Nafion* increases with the level of hydration. Exposure of *Nafion* to a humidified environment or liquid water increases the amount of water molecules associated with each sulfonic acid group. The hydrophilic nature of the ionic groups attract water molecules, which begin to solvate the ionic groups and dissociate the protons from the -SOsH (sulfonic acid) group. The dissociated protons "hop" from one acid site to another through mechanisms facilitated by the water molecules and hydrogen bonding. Upon hydration, *Nafion* phase-separates at nanometer length scales resulting in formation of an interconnected network of hydrophilic domains which allow movement of water and cations, but the membranes do not conduct electrons and minimally conduct anions due to permselectivity (charge-based exclusion).

### List of non-patent references

[1] Blackburn, P.; Gass, J.; Pinto e Vairo, F.; Farnham, K.; Atwal, H.; Macklin, S.; Klee, E.; Atwal, P. Maple Syrup Urine Disease: Mechanisms and Management. TACG 2017, Volume 10, 57-66. https://doi.org/10.2147/TACG.S125962.
[2] Kim, M. J.; Park, S.; Yang, H. J.; Shin, P.-K.; Hur, H. J.; Park, S.-J.; Lee, K.-H.; Hong, M.; Kim, J. H.; Choi, S.-W.; Lee, H.-J.; Kim, M.-S. Alleviation of Dyslipidemia via a Traditional Balanced Korean Diet Represented by a Low Glycemic and Low Cholesterol Diet in Obese Women in a Randomized Controlled Trial. Nutrients 2022, 14 (2), 235. https://doi.org/10.3390/nu14020235.
[3] Mook-Kanamori, D. O.; Römisch-Margl, W.; Kastenmüller, G.; Prehn, C.; Petersen, A. K.; Illig, T.; Gieger, C.; Wang-Sattler, R.; Meisinger, C.; Peters, A.; Adamski, J.; Suhre, K. Increased Amino Acids Levels and the Risk of Developing of Hypertriglyceridemia in a 7-Year Follow-Up. J Endocrinol Invest 2014, 37(4), 369-374. https://doi.org/10.1007/s40618-013-0044-7.
[4] Tillin, T.; Hughes, A. D.; Wang, Q.; Würtz, P.; Ala-Korpela, M.; Sattar, N.; Forouhi, N. G.; Godsland, I. F.; Eastwood, S. V.; McKeigue, P. M.; Chaturvedi, N. Diabetes Risk and Amino Acid Profiles: Cross-Sectional and Prospective Analyses of Ethnicity, Amino Acids and Diabetes in a South Asian and European Cohort from the SABRE (Southall And Brent REvisited) Study. Diabetologia 2015, 58 (5), 968-979. https://doi.org/10.1007/s00125-015-3517-8.
[5] Pitkanen, H. T.; Nykanen, T.; Knuutinen, J.; Lahti, K.; Keinanen, O.; Alen, M.; Komi, P. V.; Mero, A. A. Free Amino Acid Pool and Muscle Protein Balance after Resistance Exercise: Medicine & Science in Sports & Exercise 2003, 35 (5), 784-792. https://doi.org/10.1249/01.MSS.0000064934.51751.F9.
[6] Kitagawa, M.; Haji, S.; Amagai, T. High Serum Essential Amino Acids as a Predictor of Skeletal Muscle Depletion in Patients With Cachexia and Advanced Gastrointestinal Cancers. Nutr Clin Pract 2017, 32 (5), 645-651. https://doi.org/10.1177/0884533617724742.
[7] Hakuno, D.; Hamba, Y.; Toya, T.; Adachi, T. Plasma Amino Acid Profiling Identifies Specific Amino Acid Associations with Cardiovascular Function in Patients with Systolic Heart Failure. PLoS ONE 2015, 10 (2), e0117325. https://doi.org/10.1371/journal.pone.0117325.
[8] Puskarich, M. A.; McHugh, C.; Flott, T. L.; Karnovsky, A.; Jones, A. E.; Stringer, K. A. Serum Levels of Branched Chain Amino Acids Predict Duration of Cardiovascular Organ Failure in Septic Shock. Shock 2021, 56 (1), 65-72. https://doi.org/10.1097/SHK.0000000000001687.
[9] Mizuno, K.; Tanaka, M.; Nozaki, S.; Yamaguti, K.; Mizuma, H.; Sasabe, T.; Sugino, T.; Shirai, T.; Kataoka, Y.; Kajimoto, Y.; Kuratsune, H.; Kajimoto, O.; Watanabe, Y. Mental Fatigue-Induced Decrease in Levels of Several Plasma Amino Acids. J Neural Transm 2007, 114 (5), 555-561. https://doi.org/10.1007/s00702-006-0608-1.
[10] Nanjo, M.; Guilbault, G. G. Enzyme Electrode for L-Amino Acids and Glucose. Analytica Chimica Acta 1974, 73 (2), 367-373. https://doi.org/10.1016/S0003-2670(01)85473-8.
[11] Miškinis, J.; Ramonas, E.; Gurevičienė, V.; Razumiene, J.; Dagys, M.; Ratautas, D. Capacitance-Based Biosensor for the Measurement of Total Loss of L-Amino Acids in Human Serum during Hemodialysis. *ACS Sens.* **2022,** *7* (11), 3352-3359. https://doi.org/10.1021/acssensors.2c01342.
[12] Krebs, H. A. Chemical Composition of Blood Plasma and Serum. Annual Review of Biochemistry 1950, 19 (1), 409-430. https://doi.org/10.1146/annurev.bi.19.070150.002205.

## Claims

1. Method for making an enzyme electrode with a membrane for detection and quantification of L-amino acids comprising providing a base electrode and an enzymatic membrane **characterized in that** the enzymatic membrane is synthesized directly on the electrode surface for detection and quantification of L-amino acids, where the electrode surface is provided with gold nanoparticles to create reactive surface for optimal reaction conditions with *Nafion-based* protective membrane and the enzymatic membrane is formed using L-amino acid oxidase, bovine serum albumin and glutaraldehyde.

2. The method according to claim 1, wherein the reactive surface is synthesized using microparticles and/or nanoparticles of 2-2000 nm range in diameter made from metallic or carbon, or composite materials.

3. The method according to claim 2, where the metallic material is selected from gold, platinum, palladium, silver, nickel, antimony, titanium, ruthenium, iridium, rhodium, steel, copper or other material having similar chemical and physical properties.

4. The method according to claim 2, where the carbon material is selected from carbon particles, graphite, graphene, carbon nanotubes, fullerene-based particles or other form of carbon having similar chemical and physical properties.

5. The method according to claim 2, where the composite material is selected from organic polymers, carbon-metallic particles, bimetallic particles or other form of carbon having similar chemical and physical properties.

6. Method according to any of the preceding claims where surface of the electrode oxidizes hydrogen peroxide to oxygen in an electrochemical potential range from - 0.4 V to 0.6 V vs. standard hydrogen electrode transferring current to the electrode surface.

7. Method according to any of the preceding claims where the protective membrane is based on *Nation-,* cellulose-, polydimethylsiloxane-, poly(vinyl chloride)-, polyethylene- , Teflon-, PTFE- or other material having similar chemical and physical properties and further comprises L-amino acid oxidase mixed with bovine serum albumin or other protein having similar chemical and physical properties and crosslinked with a chemical crosslinking agent.

8. A method according to claim 7, where the crosslinking agent is glutaraldehyde, carbodiimides EDC and/or DCC, N-hydroxysuccinimide esters, such as NHS, imidoesters, maleimides, haloacetyls, pyridyl disulfides, hydrazides or other compound having similar chemical or physical properties.

9. Method according to claim 8, where the enzymatic membrane oxidizes L-amino acids producing hydrogen peroxide.

10. Enzyme electrode with a membrane for the detection and quantification of l-amino acids, where the electrode is obtained according to the method of any of the preceding claims.
